Europäisches Patentamt

European Patent Office

Office européen des brevets

(11) Publication number: **0 218 083**
**A1**

## EUROPEAN PATENT APPLICATION

(21) Application number: 86111953.5

(51) Int. Cl.4: **C12Q 1/60**

(22) Date of filing: 29.08.86

(30) Priority: 03.09.85 US 722238

(43) Date of publication of application:
15.04.87 Bulletin 87/16

(84) Designated Contracting States:
BE DE FR IT

(71) Applicant: **ABBOTT LABORATORIES**
**14th Street and Sheridan Road North St**
**North Chicago Illinois 60064(US)**

(72) Inventor: **Hefele, Jennifer E.**
**709 W. Church Street Apt. 5W**
**Champaign Illinois 61821(US)**
Inventor: **Shaffar, Mark R.**
**3365C Beacon Street No.15**
**North Chicago Illinois 60064(US)**

(74) Representative: **Modiano, Guido et al**
**MODIANO, JOSIF, PISANTY & STAUB**
**Modiano & Associati Via Meravigli, 16**
**I-20123 Milan(IT)**

(54) Stabilized cholesterol reagent and method for determining total cholesterol using the reagent.

(57) A method and stabilized reagent solutions suitable for use in the determination of total cholesterol in biological fluids. The stabilized reagents comprise three component solutions. The first solution consists essentially of a lipase in an amount greater than 7 units per milliliter, cholesterol oxidase in an amount greater than 2 units per milliliter and a peroxidase in an amount greater than 15 units per milliliter. All are present in an organic solvent and a salt solution of 0.01 to 4.0 molarity. The second component solution is a perioxidase substrate in an amount of from 1 to 200 grams per liter, a surfactant in an amount of 0.5% to 30% v/v in an aqueous solution containing an organic solvent in an amount of from 5.0% to 90% v/v. The third component solution is an aqueous solution.

EP 0 218 083 A1

Xerox Copy Centre

# STABILIZED CHOLESTEROL REAGENT AND METHOD FOR DETERMINING TOTAL CHOLESTEROL USING THE REAGENT

## BACKGROUND OF THE INVENTION

### Technical Field

The present invention relates to methods for determining cholesterol. More particularly, the invention relates to a method, and stabilized enzymatic solutions for use in the method, for determining total cholesterol in biological fluids such as serum, plasma, cerebral/spinal fluid and the like.

### Background Art

Cholesterol is present in biological fluids such as aforedescribed partially in free form and partially in bound form as a cholesterol ester. For the determination of total cholesterol, it is necessary to release the cholesterol that is bound in the cholesterol ester form. Heretofore, release of bound cholesterol has been accomplished through such methods as saponification of the cholesterol ester under alkaline conditions using alcoholic potash lye. Following the saponification, the released cholesterol can then be determined either chemically or enzymatically by known methods. Chemical determinations can be performed with or without saponification, for example, by the Liebermann-Burchard method. Also, enzymatic determinations can be performed by means using, e.g., cholesterol oxidase, cholesterol esterase or cholesterol dehydrogenase.

Methods for the determination of total cholesterol concentration in fluids such as serum or plasma typically use a single chemical treatment of cholesterol and cholesterol esters to produce a chromogenic product proportional to the total cholesterol concentration. Further refinement of chemical methods use saponification and extraction procedures, converting the cholesterol esters to free cholesterol, and then separating the cholesterol from interfering proteins. The cholesterol is then treated to produce the chromogen. Although the latter procedure is considered a reference method, it is labor intensive and does not easily lend itself to automation for the routine chemistry laboratory.

With the advent of cholesterol oxidase, enzymatic procedures became feasible. These methods can use a chemical hydrolysis of the esters, but usually use a cholesterol esterase in conjunction with surfactants and activators to cleave the ester bonds, resulting in free cholesterol. Through catalysis by cholesterol oxidase, the free cholesterol is converted to a cholestenone with the production of hydrogen peroxide. Coupling the peroxide to a chromogenic system results in color production proportional to the total cholesterol concentration. However, it is well known that cholesterol esterase procedures are plagued by incomplete ester hydrolysis. Since cholesterol oxidase cannot act on esterified cholesterol, the enzymatic procedures thus can result in inaccurate lower final concentrations than those of the reference methods which use chemical hydrolysis. Additionally, the enzymes are not stable in solution for long periods of time when exposed to the surfactants and activators required for acceptable hydrolysis during the assay. This is especially true of highly concentrated solutions which are diluted when used as the working reagents. Thus, either separate solutions for the enzymes, chromogen substrates, surfactants, and activators have been maintained, or dry reagents requiring reconstitution have been employed.

Allain, et al, Clinical Chemistry, 20 (1974), pp. 470-475, describe an enzymatic method for the determination of total serum cholesterol using a cholesterol esterase isolated from pork pancreas and rat pancreatic juice. In the disclosed method, cholesterol esterase is used on esterified cholesterol. The resulting cholesterol is treated with cholesterol oxidase to form a cholestenone and hydrogen peroxide, and the hydrogen peroxide reacted with 4-aminoantipyrine and phenol in the presence of a peroxidase to form a quinoneimine dye which is measured spectrophotometrically. In Allain, et al, one aqueous buffered solution is used to conduct the cholesterol determinations; however, the cholesterol esterases employed are known to be generally unstable in aqueous solution. Allain, et al state that the enzyme solution is relatively unstable, having a stability of only eight hours at 25°C and 24 hours at 4°C.

## SUMMARY OF THE INVENTION

The present invention is based upon the discovery of a stabilized enzymatic reagent composition, a stabilized reagent kit and a method for determining total cholesterol using the reagent kit. The reagent kit comprises three component solutions, wherein the first component solution consists essentially of a) a lipase in an amount greater than about 7 units per milliliter, b) cholesterol oxidase in an amount greater than about 2 units per milliliter, and c) a peroxidase in an amount greater than about 15 units per milliliter, elements a), b), and c) being present in an aqueous solution comprising a polyol organic solvent in an amount of from about 10% to about 75% volume/volume (v/v) and a salt solution having a molarity of from about 0.01 to about 4.0; and wherein the second component solution consists, essentially of a peroxidase substrate in an amount of from about 5 to about 200 grams per liter , and a surfactant in an amount of from about 5% to about 30% (v/v) in an aqueous solution containing an organic solvent in an amount of from about 5.0% to about 90% (v/v); and a third component solution comprising an aqueous solution.

A further aspect of the present invention provides a novel, stabilized cholesterol oleate solution which can be advantageous, utilized as a control in determinations of total cholesterol by the methods herein described.

## DETAILED DESCRIPTION OF THE INVENTION

The present invention provides a stabilized reagent kit useful in a total cholesterol assay, as described herein, which provides the unexpected advantages of stability during storage, prior to performance of an assay, as well as enabling the assay to be performed with enhanced speed. The characteristics of the stabilized composition of the invention have been assessed in studies which compared the present liquid stabilized reagents with fresh reagents. The studies show a virtual one-to-one correlation between aged liquid and fresh reagents with comparable sensitivity and precision. Moreover, providing reagents of this type in a stable form enhances color formation and thus the spectrophotometric capability of present-day instruments and methodologies, as well as improving other non-color methodologies. The reagent kit provided by the present invention is advantageous in that the kit is stable at elevated temperatures which occur during shipment. In addition, when the reagents are combined to form a working solution, the enhanced stability results in better day-to-day reproductability of the response of the reagent, thereby reducing the frequency of necessary recalibration of the reagent kit.

As more fully set forth herein, the stabilized reagent kit for total cholesterol determinations provided by the present invention is especially advantageous in a preferred method of analysis using the TDx$^R$ Analyzer, an automated analytical instrument commercially available from Abbott Laboratories, Abbott Park, Illinois. A preferred reagent kit of the present invention has also been found to offer greater assay speed in terms of the incubation time required during the assay, in contrast to methodologies of the prior art. This helps to insure the suitability and advantages of the reagent kit of the invention in assays performed on automated instrumentation, as well as in manual assays.

According to this invention, stabilization of a liquid reagent useful in the determination of total cholesterol is accomplished without resort to prior art methods such as lyophilization, preferably by preparing two separate component solutions which upon their combination into a third aqueous solution provide a stabilized, working reagent composition for determining total cholesterol by the assay methods described herein. Alternatively, the component solutions can be used in an assay separately, e.g., in an assay utilizing an oxygen electrode element, without first mixing the solutions together, or the resulting solutions can be dispensed into appropriate bottles for storage and subsequent use.

The novel reagent kit, and the components thereof provided by the invention have particular utility to improve the method of determining cholesterol which is described in U.S. Patent No. 4,495,293, the entire disclosure of which patent is incorporated herein by this reference.

The reagent kit of the invention provides the aforedescribed advantages of stability and thus can be used to improve various methods of total cholesterol measurement using a lipase with or without the addition of cholesterol esterase. Preferably, lipoprotein lipase is used having the designation E.C.#3.1.1.34 (available commercially from Toyobo Co., LTD), and is used without the additional use of various surfactants and activators.

Using the preferred lipoprotein lipase, in the performance of assays according to the invention, cholesterol ester hydrolysis has been found to occur in aqueous solutions of cholesterol esters, specifically cholesterol oleate solutions such as that prepared as described, infra, in Example 1, at concentrations up to at least 350 mg/dl; and in solutions of natural bovine serum-based cholesterol esters at concentrations of up to at least 500 mg/dl. Hydrolysis of the greater majority of serum and plasma samples has been determined to occur using a lipase concentration of 27 units per ml of sample; however, samples containing interfering compounds have resulted in more complete hydrolysis using a concentration of 136 units per ml in about 4 minutes at around 35 +/- 0.2 degrees C. Although the free cholesterol generated by lipase activity could be assayed by a number of prior art methodologies, the preferred cholesterol oxidase, horseradish peroxidase and peroxidase substrate system according to the invention as described herein has been found to be particularly and unexpectedly advantageous, also as herein described.

A "unit", as used herein in connection with amounts or concentrations of enzymes in solution is defined as the amount of enzyme which will catalyze the formation of one micromole of glycerol per minute, at pH 7 and 37 degrees C.

The preferred origin of the most preferred lipase used in the stabilized reagent kit of the invention is Pseudomonas sp., commercially available from Toyobo Co., LTD. However, other lipoprotein lipases which have been found to exhibit cholesterol esterase activity, without the necessity for the use of surfactants and activators, are:

| ENZYME SOURCE | ENZYME | SUPPLIER | E.C.# |
|---|---|---|---|
| _Chromobacterium viscosum_ | Lipase | Toyo Jozo | 3.1.1.3 |
| _Candida Cylindracea_ | Lipase | Boehringer Mannheim | 3.1.1.3 |

It is expected that the use of surfactants and activators would cause other sources of lipoprotein lipase and lipase to exhibit cholesterol esterase activity, since surfactants and activators are typically used in standard procedures for cholesterol ester hydrolysis.

The rates of hydrolysis of various cholesterol esterases are greatly increased when such esters are dispersed in micelles. Micelles are colloidal particles formed by an aggregation of small molecules. The increased rate of hydrolysis may be due to the micelle favorably orienting the ester group for attack by the enzyme. Additionally, there is some evidence that bile salts, which are surfactants, act as allosteric activators of cholesterol esterase; it is believed that they may act on lipase to prevent surface denaturation of the enzyme at the oil-water interface.

CHOLESTEROL ESTER CONTROL SOLUTION

As previously mentioned, an aspect of the present invention provides a novel, stabilized cholesterol oleate solution. This stabilized cholesterol oleate solution can be used as a non-serum based cholesterol ester solution free of serum-based interferences and having a constant cholesterol ester concentration, thereby providing a control for the purpose of determining the efficacy and stability of cholesterol assays, reagents and standard curves.

Presently, control solutions to test the efficacy of cholesterol assay kits typically contain free cholesterol, and thus do not adequately test when hydrolysis of cholesterol esters occurs. Accordingly, complete suitability of the kit for cholesterol determinations cannot always be ascertained. Also, some prior art control solutions are lyophilized and must be reconstituted; reconstitution usually results in low stability for the solution (typically one week at 2-8 degrees C). Reconstitution of another lyophilized control may thus be required, introducing the possibility of errors, and decreasing the confidence of stated cholesterol control concentrations. Additionally, serum controls may contain compounds which may interfere with different cholesterol reagents in different manners, either increasing or decreasing the reported cholesterol concentration, resulting in ambiguity as to the correct result for the method employed. For example, bilirubin can increase the reported cholesterol concentration for one method and decrease it for another. The stable liquid cholesterol ester control provided by the present invention avoids the aforementioned problems, in that it is an aqueous-based cholesterol ester control requiring no reconstitution and having long-term stability.

The components and procedure by which the cholesterol oleate solution is made are essential to the integrity of the solution. In a preferred embodiment, the solution is made as follows:

A solution of 250 milligrams per deciliter (mg/dl) cholesterol is made by heating 421 mg cholesterol oleate (Sigma, St. Louis, Mo.) and 175 mg capric acid in 10 ml Triton-100 over a boiling water solution is homogenous to 90 ml of sterile water is added, followed by heating to approximately 90 degrees C. with frequent stirring. The solution is then allowed to cool to room temperature with frequent stirring, upon which it will clear. Clarity is proportional to the cholesterol oleate concentration; thus, a 350 mg/dl solution is very turbid, whereas a 75 mg/dl solution is quite clear. After the solution is no warmer than 45 degrees C., 0.1 g of sodium azide and 0.2 g bovine serum albumin (Sigma) are added to the solution. This solution is stable at concentrations of 250 mg/dl and for at least 8 months, stored in capped glass bottles at greater than freezing temperatures.

The integrity of the cholesterol ester solution produced is described above, for comparison with solutions containing free cholesterol and fatty acids, can be determined by preparing a cholesterol reagent of the invention as described in the Examples, infra, with the exception of the inclusion of lipase. This modified reagent is then used also as described in the Examples, to test the free cholesterol content of the cholesterol ester solution. Ordinarily, the free cholesterol concentration will not exceed 15% (v/v) of the total cholesterol concentration. A 15% free cholesterol concentration is very acceptable for use in a cholesterol ester control solution; normal human serum contains approximately 70% cholesterol in an esterified form and 30% in a free cholesterol form.

The following Examples describe in more detail preferred embodiments of the invention. The Examples are intended only to illustrate and disclose, and in no way to limit the scope of the invention, which is defined solely in the claims appended hereto.

Example 1 - Solution Preparation Cholesterol Oxidase Solution

1. To 5 ml of 0.1 M K Phosphate buffer, pH 7.5, were added 2000 units of horseradish peroxidase (obtained from Sigma Chemical Co.) and 40 units Cholesterol Oxidase (from Boehringer Mannheim).

2. Lipase Solution

A 2 mg/ml concentration of lipase in distilled water was prepared, and mixed thoroughly while care was taken to prevent foaming. The resultant mixture was mixed with the Cholesterol Oxidase Solution prepared as previously described.

3. Chromogen Solution

53 g/L 3,5-dichloro-2-hydroxy benzene sulfonate (DHBS)

10 g/L 4 aminoantipyrine

7.5 g/L glycine

1 g/L azide

$4.5 \ 10^{-6}$ M fluorescein

50 ml/l surfactant (Triton x-100)

50% ethylene glycol

4. Aqueous Buffer

1.85 ml 0.1 M potassium phosphate, pH 7.5, containing 0.1% Sodium Azide and 0.01% bovine gamma globulin. Aliquots of solutions 1 and 2, and solution 3 were then introduced and mixed into solution 4, to form a stabilized working solution for a total cholesterol assay, as described in the following examples.

Example 2 -Automated Assay

An automated assay for total cholesterol in a cholesterol oleate solution (prepared as described supra) was conducted using an Abbott TDx Analyzer, substantially according to the procedure described in herein incorporated U.S. Patent No. 4,495,293. To a cuvette, 4 microliters (ul) of a sample cholesterol solution, 12.5 ul of solution 3, Example 1 and solution 4, Example 1 were added to a total volume of 1 ml. A blank reading of the fluorescence was taken and another 4 ul of serum, 12.5 ul of solution 3, Example 1, and 25 ul of solution containing lipoprotein lipase, cholesterol oxidase and horseradish peroxidase solution were introduced into the cuvette with phosphate buffer to a final volume of 2 ml. This reaction mixture was incubated at 35 degrees C. for 4.4 minutes, and a second reading of the fluorescein was taken. The decrease in fluorescence due to attenuation from the chromophore was determined to be proportional to the total cholesterol concentration.

Example 3 -Automated Assay

Another automated assay was conducted as described in Example 2, except that the sample was substantially a human serum solution. The decrease in fluorescence due to attenuation was determined to be proportional to the total cholesterol concentration.

Example 4 -Automated Assay

A further automated assay was conducted substantially in Example 2, except that the sample was a human plasma solution. The decrease in fluorescence due to attenuation was determined to be proportional to the total cholesterol concentration.

It will be appreciated that the methods for assay of cholesterol, according to the invention, as described in the preceding examples are also operable, substantially as aforedescribed, for the determination of high density lipoprotein (HDL) cholesterol. Moreover, the principles and novel reagents of the present invention can possibly be applied to the assay of substances such as human lipase; after free cholesterol is eliminated from the working sample, the rate of chromogen formation can be determined and will be proportional to the lipase activity of the sample.

According to the invention, the first component solution of the stabilized reagent kit of the invention contains a lipase, preferably a lipoprotein lipase, as previously discussed. In addition, the first solution comprises cholesterol oxidase and a peroxidase. It is to be appreciated that the amounts of these constituents can vary widely in the ranges specified herein, and are limited only in their upper concentration limits by their solubility in the solution, which also comprises a polyol organic solvent and a salt solution. Moreover, it will be appreciated that the peroxidase can be selected from any conventional peroxidase known in the art, and can, for example, be a microperoxidase or similar variant substance which functions as a peroxidase.

The organic solvent can be selected from any number of suitable compounds, and can comprise, for example, dimethyl sulfoxide, ethylene glycol, butylene glycol, polyethylene glycol, glycerol, propylene glycol, dimethyl formamide, tetrahydrofuran, methanol, ethanol, propanol, butane or pentanediol, or mixtures of the foregoing or of others. The salt solution of the first and second component solutions can be; e.g., sodium or potassium phosphate, ammonium sulfate, sodium acetate, potassium acetate, sodium sulfate and the like compounds, or mixtures thereof. As the organic solvent in the first component solution, glycerol, polyethylene glycol, ethylene glycol, mannitol, sorbitol, propylene glycol, or mixtures thereof, and the like, can be used. As the surfactant, use can be made of well-known substances such as Triton x-100, or the like.

The peroxidase substrate used, it will be appreciated, can be selected from various substances capable of producing a detectable response in the presence of a peroxidase, such as a chromogenic, fluorescent or chemiluminescent response. Examples of suitable chromogenic substrates include o-dianisidine, phenol/aminoantipyrene, DHBS/aminoantipyrene, 5-amino salicylic acid, 3-amino-9-ethylcarbazole, 2,2' azino-di(3 ethyl-benz-thiazoline-6-sulfonate), benzidine and 3-3' diaminobenzidine.

Examples of suitable fluorescent peroxidase substrates include, without limitation homovanillic acid, ortho-phenylenediamine, scopoletin, diacetyldichlorofluorescein, p-hydroxphenylacetic acid, tyramine, tyrosine, and 3,4 dihydroxyphenylacetic acid.

Examples of suitable chemiluminescent substrates include components such as bis (2,4-dinitrophenyl) oxalate, bis (3-trifluoromethyl-4-nitrophenyl) oxalate, bis-(triphenylacetic) oxalic anhydride, luminol, oxalyl chloride, and indoyl peroxide with suitable fluorophores.

In order to more fully illustrate the advantages of the present invention, a preferred cholesterol assay in accordance with the invention; as described in Example 2 was compared to prior art methods by assaying clinical specimens obtained from persons suspected of having elevated cholesterol levels. The linear regression results are set forth in the following Table 1, and subsequent Table 2 sets forth further comparisons.

## TABLE 1

| The preferred cholesterol assay performed on the TDx Analyzer, versus | Total Number of Samples Analyzed | y-Intercept | Slope | Correlation Coefficient |
|---|---|---|---|---|
| A-Gent[R] Cholesterol performed on the Abbott ABA 200[R] analyzer | 200 | 3.9 | 0.988 | 0.982 |
| Sigma Cholesterol performed on the Abbott Analyzer | 200 | 17.0 | 0.966 | 0.991 |
| Boehringer Mannheim High Performance Cholesterol performed on the Gilford Autocarousel Analyzer | 215 | -5.3 | 1.02 | 0.993 |
| Dupont cholesterol performed on the DuPont ACA Analyzer | 217 | -7.4 | 1.04 | 1.00 |
| Liebermann-Burchard performed on the Technicon Autoanalyzer II | 120 | -12.4 | 1.10 | 0.995 |

TABLE 2

| | Assay According to Present Invention | A-GENT (Abbott Laboratories) | Sigma | Boehringer Mannheim High Performance |
|---|---|---|---|---|
| Measures | Cholesterol | Cholesterol | Cholesterol | Cholesterol |
| Principle | Coupled enzyme assay | Coupled enzyme assay | Coupled enzyme assay | Coupled enzyme assay |
| Reaction System | Lipolytic enzyme Cholesterol oxidase Horseradish peroxidase | Cholesterol esterase Cholesterol oxidase Horseradish peroxidase | Cholesterol esterase Cholesterol oxidase Horseradish peroxidase | Cholesterol esterase Cholesterol oxidase Horseradish peroxidase |
| Detection Method | Fluorescence | Absorbance | Absorbance | Absorbance |
| Sample Form | Serum or plasma | Serum or plasma | Serum or plasma | Serum or plasma |
| Sample Volume | 8.0 microliters (ul) | 2.5 ul | 2.5 ul | 2.5 ul |
| Reagent Volume | 2 ml | .250 ml | .250 ml | .250 ml |
| Incubation Temperature | 35 degrees C | 37 degrees C | 37 degrees C | 37 degrees C |
| Incubation Time | 4.3 minutes | 10 minutes | 10 minutes | 2 minutes |
| Reagent & Form | Liquid (ready-to-use) | Dry Package (requires reconstitution) | Dry Package (requires reconstitution) | Dry Package (requires re-constitution) |
| Liquid Reagent Stability | 18 mo. room temperature | 1 week 2-8 degrees C | N/A | 1 mo 2-8 degrees C |

The improved stability of the novel reagent kit of the invention was demonstrated by observing a consistent response of the reagent prepared as in Example 2. Tables 3 and 4 show the percent transmittance as a measure of the response of the reagent over the life of the reagent.

0 218 083

## TABLE 3

### STABILITY OF THE REAGENT KIT WHEN STORED AT 2-8°

| Time | % Transmittance of O mg/dl Calibrator | % Transmittance of 400 mg/dl Calibrator | Cholesterol Concentration of Serum Control |
|---|---|---|---|
| 0 | 95 | 14 | 270 |
| 1 mo. | 95 | 18 | 270 |
| 4 mo. | 96 | 16 | 272 |
| 8 mo. | 97 | 10 | -- |
| 16 mo. | 92 | 19 | 275 |

0 218 083

## TABLE 4

STABILITY OF THE REAGENT KIT WHEN STORED AT ROOM TEMPERATURE

| Time | % Transmittance of 0 mg/dl Calibrator | % Transmittance of 400 mg/dl Calibrator | Cholesterol Concentration of Serum Control |
|------|---------------------------------------|-----------------------------------------|--------------------------------------------|
| 0 | 96 | 13 | -- |
| 1 mo. | 96 | 18 | 269 |
| 2 mo. | 98 | 14 | 275 |
| 4 mo. | 98 | 19 | 272 |
| 8 mo. | 94 | 16 | -- |

It is apparent that various modifications and variations of the specific compositions, methods and techniques of the invention as described herein can be envisioned by one of ordinary skill in the art, without departing from the spirit and scope of the invention, as set forth solely by the following claims.

## Claims

1. A stabilized reagent kit useful in a determination of total cholesterol, which reagent kit comprises three component solutions, wherein the first component solution consists essentially of: a) a lipase in an amount greater than about 7 units per milliliter, b) cholesterol oxidase in an amount greater than about 2 units per milliliter and c) a peroxidase in an amount greater than about 15 units per milliliter, elements a), b) and c) being present in an aqueous solution comprising an organic solvent in an amount of from about 10% to about 75% (v/v) and a salt solution having a molarity of from about 0.01 to about 4.0; wherein the second component solution consists essentially of a peroxidase substrate in an amount of from about 1 to about 200 grams per liter and a surfactant in an amount of from about 0.5% to about 30% (v/v), in an aqueous solution containing an organic solvent in an amount of from about 5.0% to about 90% (v/v); and wherein the third component solution comprises an aqueous solution.

2. The reagent kit of claim 1, wherein the first and second component solutions are combined.

3. A method for making a stabilized cholesterol reagent, comprising adding the first and second component solutions of Claim 1 to the third component solution at claim 1, whereby the pH of the resulting mixture is between about 4 and about 9.

4. A stabilized cholesterol reagent produced by the method of claim 3.

5. The reagent kit of claim 1, wherein the first component solution consists essentially of, in addition, a cholesterol esterase.

6. In a method for determining total cholesterol which comprises mixing a reagent comprising cholesterol oxidase, peroxidase, a peroxidase substrate and a lipase, with a cholesterol-containing fluid, incubating, and measuring a detectable response produced by a reaction product formed from the peroxidase substrate, the improvement wherein the reagent consists essentially of the stabilized reagent of claim 4.

7. The stabilized cholesterol reagent kit of claim 1 wherein the reagent kit is stable under elevated temperatures and for extended periods of time when refrigerated.

8. The stabilized cholesterol reagent kit of claim 2 wherein the reagent kit is stable for extended periods of time when refrigerated, thereby providing a stabilized calibration response.

9. The reagent kit of claim 1, wherein the peroxidase substrate forms a fluorescent product.

10. The reagent kit of claim 1, wherein the peroxidase substrate forms a chemiluminesent or bioluminescent product.

European Patent Office

# EUROPEAN SEARCH REPORT

Application number

EP 86 11 1953

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| X | CHEMICAL ABSTRACTS, vol. 89, no. 21, 20th November 1978, page 277, abstract no. 175999x, Columbus, Ohio, US; & JP-A-78 81 194 (TOYOBO CO. LTD.) 18-07-1978 * Whole abstract * | 1,5,6 | C 12 Q 1/60 |
| Y | FR-A-2 379 815 (EASTMAN KODAK CO.) * Claims 1-4,6-8; page 4, lines 3-33; page 7, lines 26-40 * | 1,6 | |
| Y | US-A-4 378 429 (I.E. MODROVICH) * Abstract; claims 1,8,21,45,62,63 * | 1,3,6 | |
| P,D Y | EP-A-0 155 330 (ABBOTT LABORATORIES) * Page 27, example 3 * | 1,3,6 | TECHNICAL FIELDS SEARCHED (Int. Cl.4)  C 12 Q |
| A | DE-A-3 432 348 (REANAL FINOMVEGYSZERGYAR) * Pages 27,28 * | 1 | |
| A | EP-A-0 091 026 (I.E. MODROVICH) * Claim 11 * | 1,6 | |
| A | US-A-4 414 326 (J.M. GOLDBERG) * Abstract; claims 1,5,7 * | 1,6 | |
|  | ---     -/- | | |

The present search report has been drawn up for all claims

| Place of search | Date of completion of the search | Examiner |
|---|---|---|
| THE HAGUE | 02-12-1986 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on. or after the filing date
D : document cited in the application
L : document cited for other reasons

& : member of the same patent family, corresponding document

EPO Form 1503 03 82

European Patent
Office

**EUROPEAN SEARCH REPORT**

Application number

EP  86 11 1953

Page  2

## DOCUMENTS CONSIDERED TO BE RELEVANT

| Category | Citation of document with indication. where appropriate, of relevant passages | Relevant to claim | CLASSIFICATION OF THE APPLICATION (Int. Cl.4) |
|---|---|---|---|
| A | EP-A-0 084 684  (BOEHRINGER MANNHEIM GmbH) * Claims;  page 1; page 3, lines 1-6 * | 1,3 | |
| | ----- | | |

TECHNICAL FIELDS SEARCHED (Int. Cl.4)

The present search report has been drawn up for all claims

| Place of search THE HAGUE | Date of completion of the search 02-12-1986 | Examiner MEYLAERTS H. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document